# EUROPEAN PATENT SPECIFICATION

(11) **EP 1 507 869 B1**
(45) Date of publication and mention of the grant of the patent: **20.01.2010**
(21) Application number: 03756374.9
(22) Date of filing: 29.05.2003
(51) Int. Cl.: C12Q 1/34, G01N 33/542

(54) **IMPROVED RECEPTOR DETECTION**
VERBESSERTER REZEPTORNACHWEIS
IMPROVED RECEPTOR DETECTION

(30) Priority: 29.05.2002 US 384060 P
(43) Date of publication of application: 23.02.2005
(73) Proprietor: DiscoveRx Corporation, Fremont, CA 94538 (US)
(72) Inventor: NAQVI, Tabassum, Fremont, CA 94538 (US); ROUHANI, Riaz, Concord, CA 94521 (US); SINGH, Rajendra, San Jose, CA 95135 (US)
(74) Representative: Sexton, Jane Helen
(86) International application number: PCT/US2003/017428
(87) International publication number: WO 2003/102154

(56) References cited:
- WO-A-03/093786
- US-A- 4 708 929
- US-A- 5 223 393
- US-A- 5 976 857
- HENDERSON D R ET AL: "CEDIA TM, A NEW HOMOGENEOUS IMMUNOASSAY SYSTEM" CLINICAL CHEMISTRY, AMERICAN ASSOCIATION FOR CLINICAL CHEMISTRY, WASHINGTON, DC, US, vol. 32, no. 9, 1 September 1986 (1986-09-01), pages 1637-1641, XP000653195 ISSN: 0009-9147

## Description

### BACKGROUND OF THE INVENTION

### FIELD OF THE INVENTION

The invention relates generally to detection of protein receptors using labeled ligands; the protein receptors being either an enzyme or a membrane receptor.

### BACKGROUND INFORMATION

Naturally occurring receptors are a key element in the physiology of cells. By receptors are intended enzymes and complex forming proteins, e.g.membrane receptors, rather than antibodies or fragments thereof, particularly polyvalent fragments, e.g. F(ab')₂. There is a substantial difference in the function of the receptors and the antibodies. The receptors, particularly the enzymes, do not have high affinity for their ligands, in the case of the enzymes, their substrates are the ligands. Therefore, in many cases the affinities are substantially lower for the ligand binding with the receptor as compared to the antibody. In the case of the enzyme, the substrate must be bound, converted to product and then expelled from the active site to allow for new substrate to bind. In the case of the receptor, upon binding of the ligand the receptor can undergo a variety of conformational and chemical changes. In order to continue to be active, the ligand must be released or the complex endocytosed, where the ligand may be degraded and the receptor returned to the cell membrane or the complex degraded. Identifying binding to the receptor or providing a competitive ligand for assaying for drugs binding to the receptor remains of great interest.

The small fragment of β-galactosidase known as the enzyme donor ("ED") has found extensive use as a label in diagnostic assays. When bound to another molecule and complexed with the large fragment of β-galactosidase, an active enzyme is produced with a high turnover rate, acting on substrates that can give an optical signal, e.g. fluorescent, absorbent or chemiluminescent signal. The literature has indicated that the turnover rate observed substantially deteriorates as one reduces the size of the ED. For the most part, an ED of about 90 amino acids has been used commercially, where the N- and C-terminal amino acids are functionalized, so that there will be two ligands, one at each end. When binding to antibody, the two ligands provide for a very high avidity due to the ligand and the antibodies both having two binding sites. The resulting complex is highly favored. By contrast, with receptors, the receptor will usually have a single binding site, so that the presence of two ligands bound to the ED will not provide the same avidity as observed with antibodies.

Because of the reduced binding affinity observed with receptors, any label bound to the ligand must not reduce the binding affinity of the ligand further. The label should not affect the conformation of the ligand, the site of binding, and the contacts of the ligand with the surface of the receptor, while at the same time be available for efficient binding to the EA to provide a high turnover rate. Even where there may be a ligand developed having a higher affinity than the natural ligand, many of the same considerations apply.

There is, therefore, an interest in developing reagents that permit the sensitive detection of receptors, that allow for competitive assays, and that may be readily produced as conjugates or fused proteins.

### RELEVANT LITERATURE

U.S. Patent nos. 4,378,428; 4,708,929; 5,037,735; 5,106,950; 5,362,625; 5,464,747; 5,604,091; 5,643,734;and PCT application nos. WO96/19732; and WO98/06648 describe assays using complementation of enzyme fragments. WO 00/039348, as indicated above, describes a protease assay where the marker is a β-galactosidase fragment fused to a protein having a specific protease cleavage site.
There are numerous other references concerned with the use of β-galactosidase fragments in assay systems. The following are illustrative. Douglas, et al., Proc. Natl. Acad. Sci. USA 1984, 81:3983-7 describes the fusion protein of ATP-2 and lacZ. WO92/03559 describes a fusion protein employing α-complementation of β-galactosidase for measuring proteinases. WO01/0214 describes protein folding and/or solubility assessed by structural complementation using the α-peptide of β-galactosidase as a fusion protein. WO01/60840 describes fusion proteins including a fusion protein comprising an enzyme donor β-galactosidase for measuring protein folding and solubility. Homma, et al., Biochem. Biophys. Res. Commun., 1995, 215, 452-8 describes the effect of α-fragments of β-galactosidase on the stability of fusion proteins. Abbas-Terki, et al., Eur. J. Biochem. 1999, 266, 517-23 describes α-complemented β-galactosidase as an *in vivo* model susbtrate for the molecular chaperone heat-shock protein in yeast. Miller, et al., Gene, 1984, 29, 247-50 describe a quantitative β-galactosidase α-complementation assay for fusion proteins containing human insulin β-chain peptides. Thomas and Kunkel, Proc. Natl. Acad. Sci. USA, 1993, 90, 7744-8 describe an ED containing plasmid to measure mutation rate.

### SUMMARY OF THE INVENTION

In one embodiment, the invention provides a method for determining a binding event of a protein receptor with a ligand, wherein said protein receptor is other than an antibody or polyvalent fragment thereof and is either an enzyme or is a membrane receptor wherein the membrane receptor is a component of an intact cell or of a lysed cell, said method comprising:
employing as enzyme donor (ED) a β-galactosidase fragment of from 36 to 50 amino acids;
combining a conjugate of said ED and of a ligand complementary to said protein
   receptor with said protein receptor; and
measuring the amount of complex formation of said ED with an enzyme acceptor fragment (EA) by means of a detectable product provided by a substrate.

In a preferred embodiment of the method of the invention, the protein receptor is an enzyme. In another preferred embodiment of the method of the invention, the protein receptor is a membrane receptor.

Also disclosed are reagents of the short β-galactosidase fragment, the enzyme donor fragment ("ED"), and a ligand for a receptor (enzymes and complex forming proteins other than antibodies and fragments thereof, particularly polyvalent fragments) are provided, where the ED provides for low interference with binding of the ligand to the receptor and efficient complexing with the large β-galactosidase fragment, the enzyme acceptor fragment ("EA"), for a high turnover rate. Of particular interest are assays for enzymes, where greater activity is observed for the smaller EDs. The reagents are conjugates or fused proteins. The reagents may be used for detecting the receptors or in competitive assays.

### BRIEF DESCRIPTION OF THE FIGURES

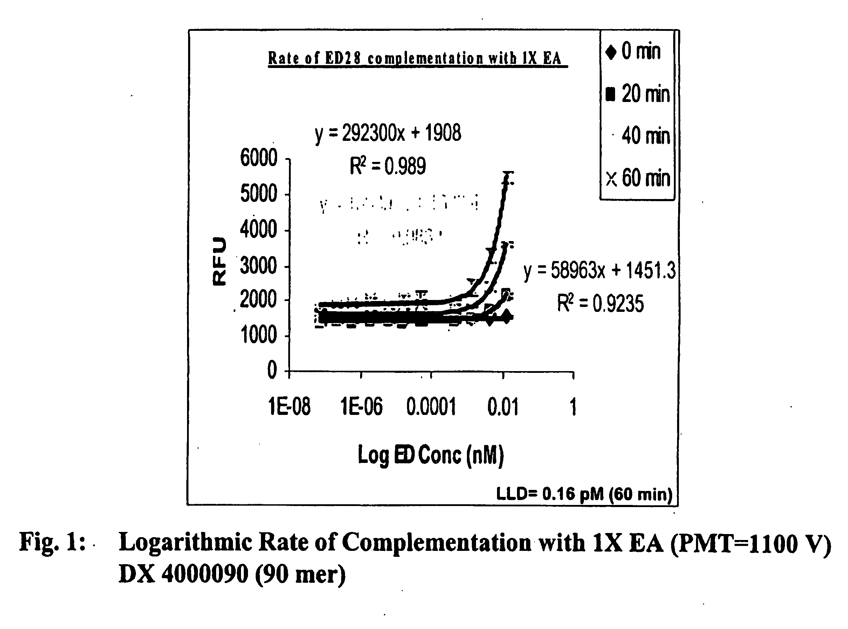

Fig. 1 is a graph of the rate of complementation of the 90 mer DX 400090 with EA at different time course and at different concentrations of ED at a concentration of 1x EA (0.18 mg/ml) with the lowest limit of detection ("LLD") indicated;

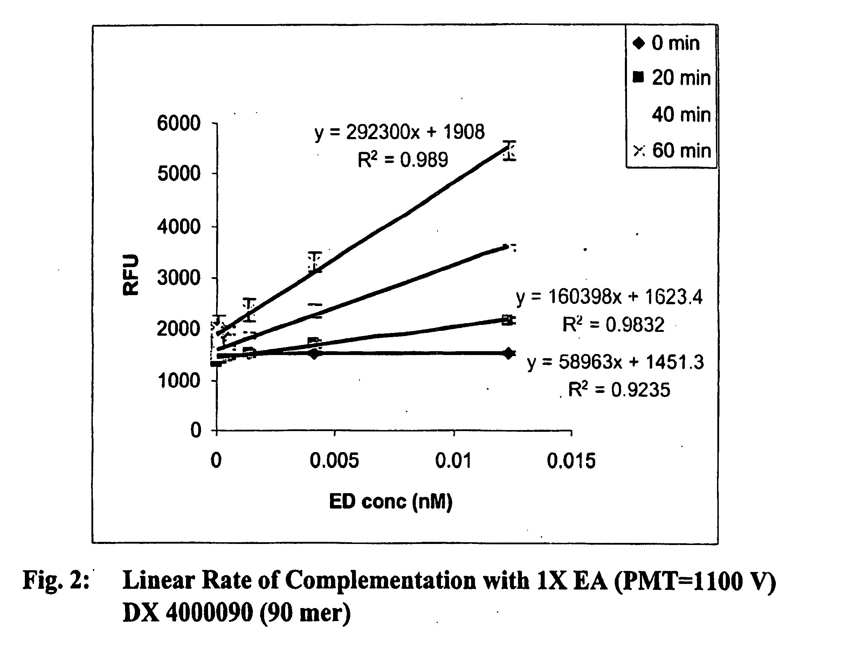

Fig. 2 is a partial repeat of the rate determinations of Fig. 1 over a smaller concentration range;

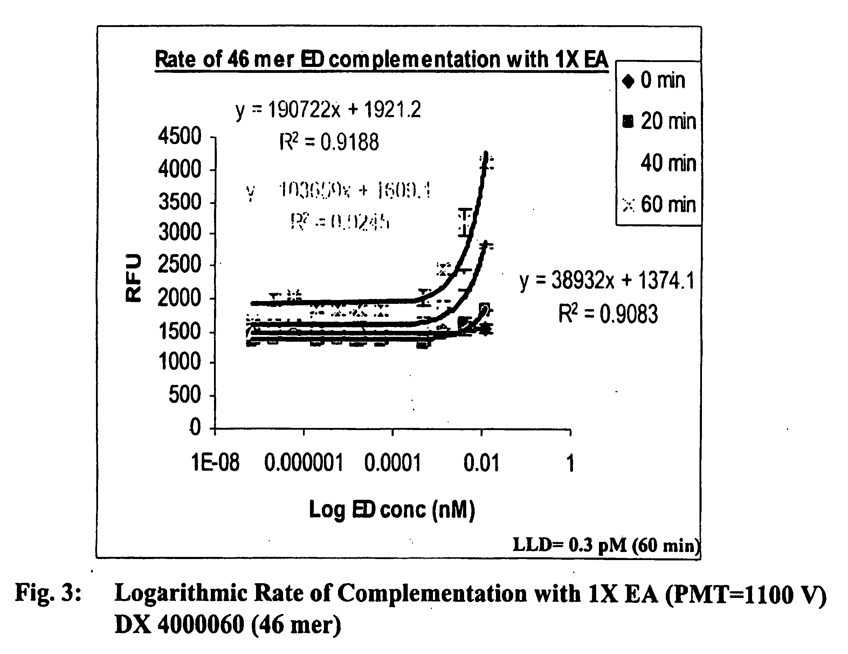

Fig. 3 is a repeat of the determination of Fig. 1 where the 45+1 mer DX 400060 is employed;

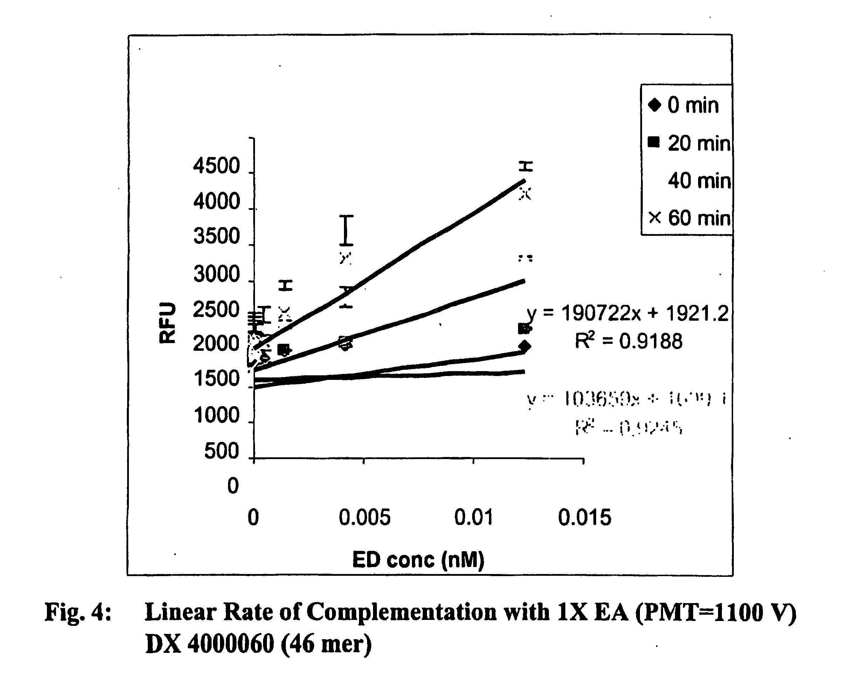

Fig. 4 is a repeat of the rate determinations of Fig. 2 where the 45+1 mer DX 400060 is employed;

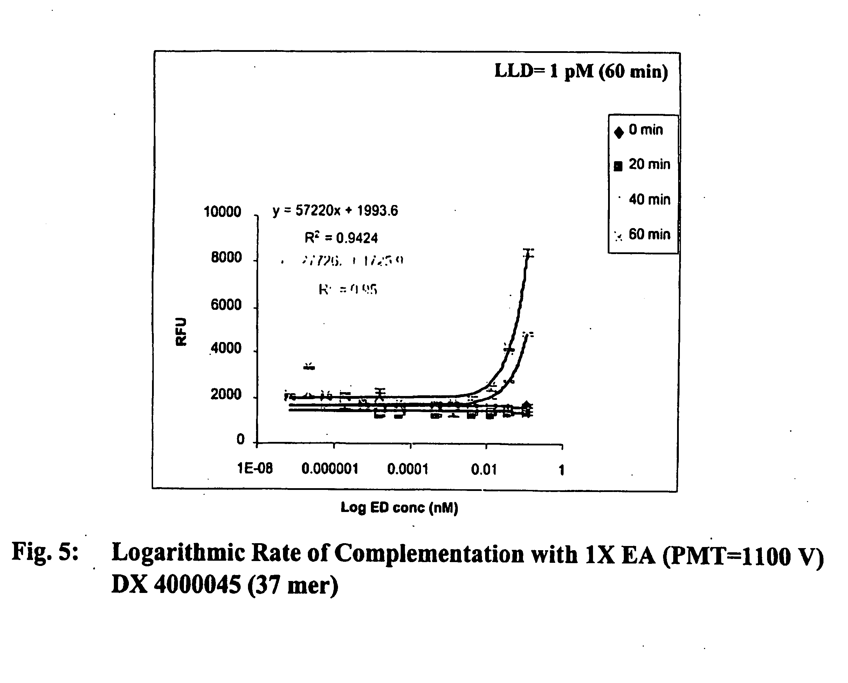

Fig. 5 is a repeat of the determinations of Fig. 1, where the 37 mer DX 400045 is employed;

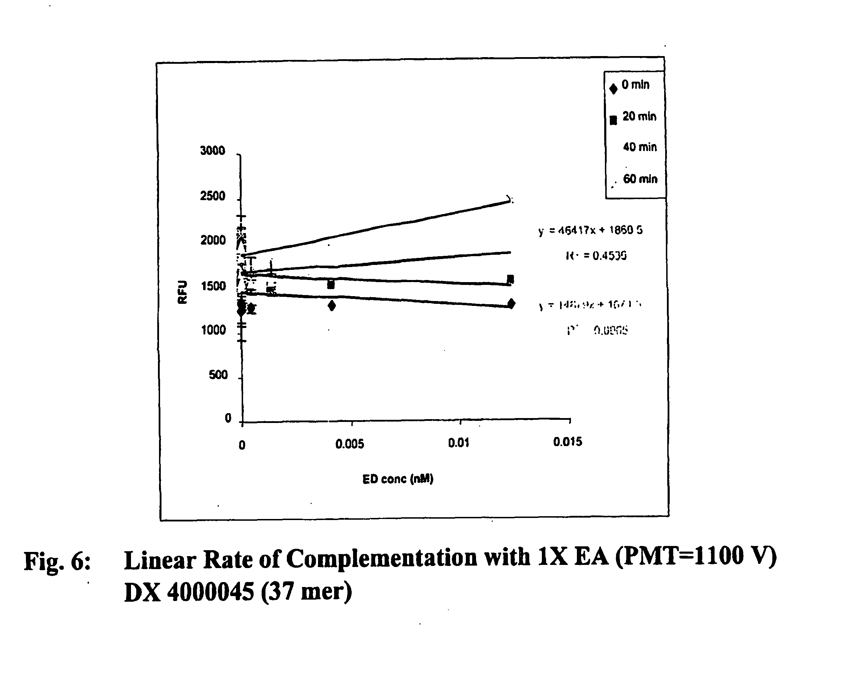

Fig.6 is a repeat of the determinations of Fig. 2, where the 37 mer DX 400045 is employed;

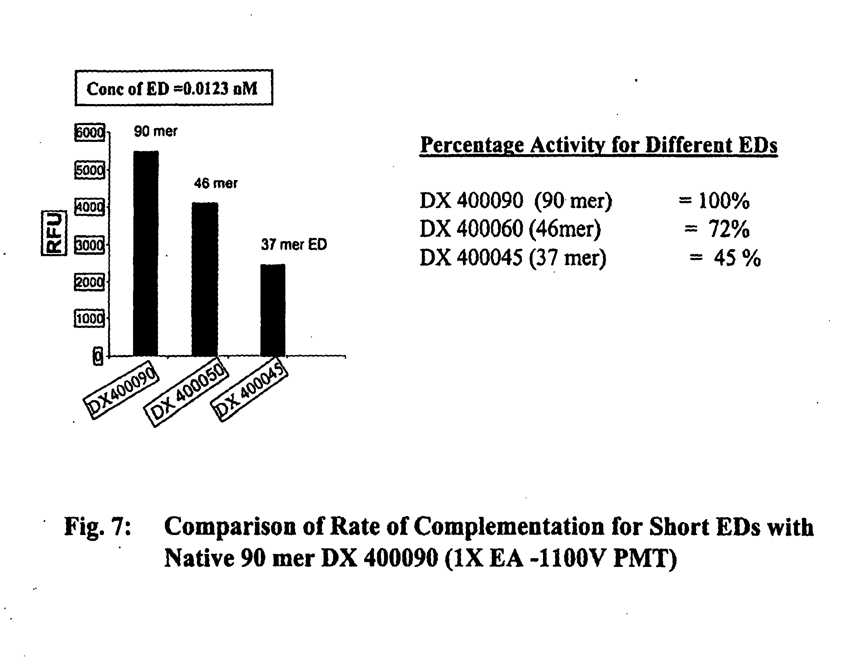

Fig. 7 is a bar graph comparing the results of the previous figures;

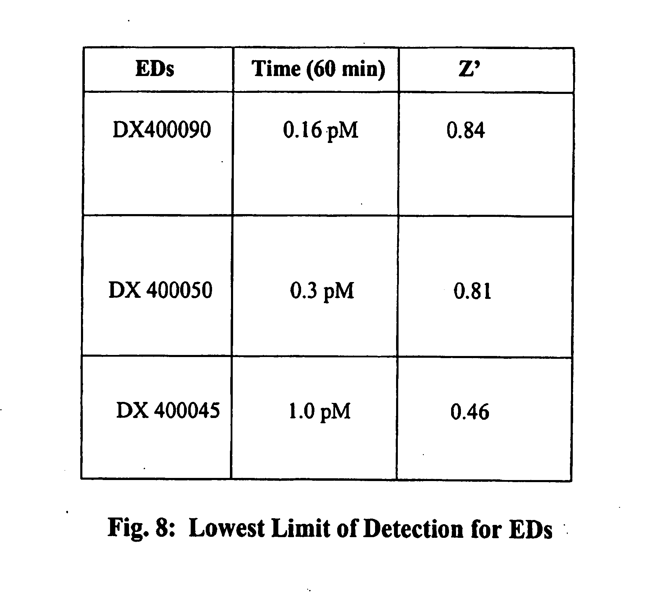

Fig. 8 is a table showing the LLDs at 60 min, where Z' is a level of confidence measure based on standard deviation;

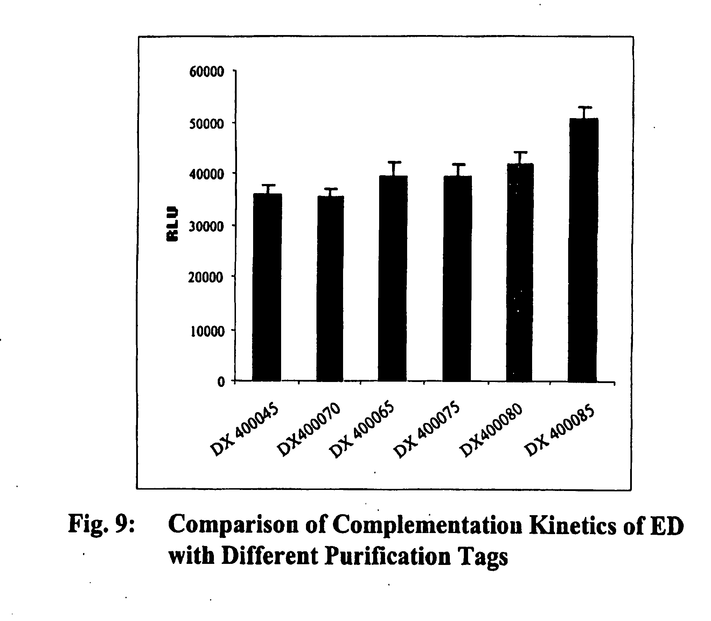

Fig. 9 is a bar graph comparing the complementation kinetics of the different EDs with the different extensions allowing for purification; and
Fig. 10 is a graph of the assays with two different ED-staurosporine conjugates at varying concentrations, indicting that the shorter ED46+2-2C-SS has a higher affinity for the enzyme PKC-alpha.
Fig. 11 is a graph of p38-MAP Kinase Standard Curve with SB-202190.

### DETAILED DESCRIPTION OF THE INVENTION

Reagents and assays are provided for measuring the availability of receptor binding sites. The reagents are conjugates or fusion proteins, where the active entity is the small fragment of β-galactosidase, the enzyme donor ("ED") and the natural ligand or a mimetic analog. Where the ligand is a polypeptide, the reagent may be a fusion protein, while where the ligand is other than a polypeptide, the reagent will be a conjugate, having from one to two ligands. (By conjugate is intended a polypeptide linked through a covalent bond from an amino acid to a ligand that is other than an amino acid or polypeptide.) The assays are performed by having in an assay medium the reagent comprising the ED, the receptor, the large β-galactosidase fragment (the enzyme acceptor ("EA")), and substrate. The substrate provides a detectable product, where the detectable product is measured as an indication of the presence of the receptor.

The ED may be obtained from any source and will have from about 35 to 50 amino acids total for the active entity, generally having from 36, usually 37, to 50 amino acids and for linking may have one to two additional cysteines. EDs are extensively described in the aforementioned reference, U.S. Patent no. 4,708,929, (see in particular section 6). Thus, the β-galactosidase sequence will normally be obtained from a unicellular microorganism, particularly *E. coli.* However, not more than 3 of the amino acids may be substituted for the naturally occurring amino acids. For the most part, conservative substitutions are involved, where the non-polar aliphatic amino acids, such as G, A, V, L, and I may be substituted one for the other, the non-charged polar amino acids, such as C, M, S, T, N, and Q may be substituted one for the other, the charged amino acids may be substituted one for the other of the same charge, i.e. K and R; and D and E; and the aromatic amino acids may be substituted one for the other, F, W, and Y. Generally the active portion of the molecule will not be changed, except that it may be joined at either of its termini to a compound of interest, particularly a polypeptide. In addition one or two cysteine amino acids may be added proximal to the termini, within 6, usually 3, amino acids of the terminus. The number for the mer refers to the amino acids naturally present in the ED. The ED may be joined by an amino acid linker to a polypeptide of interest, generally of from about 1 - 10 amino acids, usually naturally occurring amino acids. The linker will ordinarily not be the natural sequence of the β-galactosidase that follows the ED, so that the amino acid(s) following the active sequence will be other than the amino acid(s) that have found exemplification in the literature. Numerous sequences are set forth in '929, which may be used herein, and when other than a fused protein, may have N- or C-proximal cysteine for conjugation to a ligand.

The cysteine(s) serve as sites for linking by employing a ligand that forms a covalent bond with a thiol. Various functionalities can react with a thiol, including activated olefins, e.g. having an acryloyl functionality, active halogen or pseudohalogen, e.g. halomethylcarbonyl, thiol to form a disulfide, etc. Thus, one can have from one to two ligands depending upon the number of cysteines. The reacting functionality may be joined directly to the ligand or through a linking chain, usually an innocuous linking chain, of from about 1 to 12 atoms, where the linker may provide some functionality, such as hydrophilicity, solubility, sequestration capability, etc.

### Sequences of interest for the ED nclude:

SLAVVLQRRDWENPGVTQLNKLAAHPPFASWRNSEE**A (37 mer) (SEQ ID:NO 1)**
SLAWLQRRDWENPGVTQLNKLAAHPPFASWRNSEEARTDCPSQQL (46 mer) **(SEQ ID:NO 6)**

For linking with non-polypeptide ligands, the EDs of particular interest are those having the ED sequence while including from one to two cysteines, each cysteine being within 6, usually 3, amino acids of the terminus of the ED, including being at the terminus. These EDs of fewer than 51 amino acids can be readily and accurately synthesized by known techniques, particularly automated equipment generally available today, e.g. from Applied Biosystems Inc. Amersham, etc. The newly synthesized ED may then be released from the support or may be conjugated with the ligand while on the support followed by release of the conjugate. An ED of particular interest has 45 amino acids of the β-galactosidase N-terminal proximal region and 1-2 cysteines, each cysteine within 4 amino acids of the N- and C-terminus, respectively.

The ligands of interest include naturally occurring and synthetic ligands, generally ranging in molecular weight from about 125 to 500,000 Dal. For small organic molecules, particularly synthetic organic molecules, the molecular weight will generally be in the range of about 125 to 2000 Dal, for oligomers, e.g. polypeptides and polyketides, from about 250 to 5,000, while for polymers, e.g. proteins and polysaccharides, the molecular weights will generally be in the range of 2000 Dal to about 500 kDal, usually not more than about 250 kDal. The ligands may be acyclic or cyclic, carbocyclic or heterocyclic. Generally, the ligands, particularly enzyme inhibitors, will have a binding affinity of at least about 10⁻⁷ M, more usually at least about 10⁻⁸ M, and frequently higher. The ligands may be naturally occurring ligands, mimetic ligands, agonists, antagonists, etc.

The non-polymeric organic molecules may be naturally occurring or synthetic and include naturally occurring and synthetic drugs, steroids, polyketides, amino acids, lipids, sugars, rare naturally occurring amino acids, e.g. D-amino acids, etc. Illustrative ligands include steroids, nucleotides, e.g. triphosphates, hormones, drugs, enzyme substrates, etc. The polymeric ligands may be naturally occurring or synthetic and include hormones, polysaccharides, particularly associated with glycoproteins, interleukins, colony stimulating factors, interferons, morphogens, etc., where these proteins will usually be neither enzymes nor membrane receptors.

The receptors are proteins that bind another molecule, the ligand, which may be another protein or other molecule. For the most part the receptors are monovalent binding proteins, although monovalent proteins such as Fab fragments will normally not be included in the family of receptors. Binding events include enzymes binding to their respective substrates or antagonists, complex formation between a protein ligand, e.g. hormone and its membrane receptor, complex formation between transcription factors, components of a functional structure, such as a ribosome, spliceosome, transcription initiation complex, etc., integrin and adhesion molecule binding, as well as other naturally occurring events involving complex formation between one or more proteins and a protein and a non-protein. The membrane receptors may be cellular membrane or internal membrane, e.g. nuclear, receptors.

Of the protein categories of interest, transcription factors, inhibitors, regulatory factors, enzymes, membrane proteins, structural proteins, integrins, adhesion molecules, and proteins complexing with any of these proteins, are of interest. Specific proteins include enzymes, such as the hydrolases exemplified by amide cleaving peptidases, such as caspases, thrombin, plasminogen, tissue plasminogen activator, cathepsins, dipeptidyl peptidases, prostate specific antigen, elastase, collagenase, exopeptidases, endopeptidases, aminopeptidase, metalloproteinases, including both the serine/threonine proteases and the tyrosine proteases,; hydrolases such as acetylcholinesterase, saccharidases, lipases, acylases, ATP cyclohydrolase, cerebrosidases, ATPase, sphingomyelinases, phosphatases, phosphodiesterases, nucleases, both endo- and exonucleases,; oxidoreductases, such as the cytochrome proteins, the dehydrogenases, such as NAD dependent dehydrogenases, xanthine dehyrogenase, dihydroorotate dehydrogenase, aldehyde and alcohol dehydrogenase, aromatase,; the reductases, such as aldose reductase, HMG-CoA reductase, trypanothione reductase, etc., and other oxidoreductases, such as peroxidases, such as myeloperoxidase, glutathione peroxidase, etc., oxidases, such as monoamine oxidase, myeloperoxidases, and other enzymes within the class, such as NO synthase, thioredoxin reductase, dopamine β-hydroxylase, superoxide dismutase, nox-1 oxygenase, etc.; and other enzymes of other classes, such as the transaminase, GABA transaminase, the synthases, β-ketoacyl carrier protein synthase, thymidylate synthase, synthatases, such as the amino acid tRNA synthatase, transferases, such as enol-pyruvyl transferase, glycinamide ribonucleotide transformylase, COX-1 and -2, adenosine deaminase.

Kinases are of great significance, such as tyrosine kinases, the MAP kinases, the cyclin dependent kinases, GTP kinases, ser/thr kinases, Chk1 and 2, etc.

Also of interest are enzyme inhibitors, such as α₁-antitrypsin, antithrombin, cyclophilin inhibitors, proteasome inhibitors, etc.

Neuronal proteins, such as β-amyloid, TNF, prion, APP, transporters, e.g. dopamine transporter, receptors, such as NMDA receptors, AMDA receptors, dopamine receptors, channels, etc.

Another class of proteins is the transcription factors and their inhibitors or regulatory proteins, such as Adr Ace, Amt, AP, Atf, Att, Baf, Bm, Btf, C Ebp, C Jun, C Ets, CREB, CF, Chop, DP, E2F, Elk, Gata, Hnf, Iii A-H, Irf, NY Y, Otf, NFκB, NF-AT, Oct-1, Pea, Pit, PU, S, SP, Stat, Tef, TFIII, TFIIII, Ubf and Usf, while the inhibitors include Erk, IκB, LIF, Smad, RANTES, Tdg, etc., as well as other proteins associated with pathways that induce transcription factor synthesis, activation or inhibition.

In some instances, housekeeping proteins will be of interest, such as the proteins involved in the tricarboxylic acid cycle, the Krebs cycle, glycogenesis, etc.

The assays may be intra- or extracellular. When intracellular, the cell will be required to have functional EA present in the cell, as a result of expression of the EA in the cell or introduction of EA by having a cell permeable EA added to the cell culture. In addition, a cell permeable substrate is employed, such substrates being disclosed in U.S. Patent nos. 5,208,148 and 5,576,424. Alternatively, the cells may be lysed and the lysate assayed directly or after enhancement of the concentration of the receptor. Other assays may be performed on other than cellular sources, such as competitive assays, where one is interested in the binding of a ligand to the receptor.

The assay will normally be performed in an aqueous buffered medium selected for obtaining the desired binding affinity of the target(s) for the ED-conjugate. The pH of the medium will generally be in the range of about 3 - 11, more usually in the range of about 5 - 9. The volume of the assay composition is primarily one of convenience, taking into consideration the cost of the reagents, the available equipment, the number of assays to be performed, the sensitivity of detection, and the like. The assay may be performed in microtiter plate wells, ranging from 96 well plates to about 1536 well plates. The volumes may be from about 10 nl to 1ml, usually varying from about 50 nl to 500 µl. The concentration of the reagents, the ED-conjugate and the EA, will vary with the concentration range of interest of the protein-binding candidate. The concentrations of the reagents, ED-conjugate, EA and target protein may be determined empirically to optimize the sensitivity of the assay for the particular protein. For competitive assays, generally, the concentration of the ED-conjugate will be in the range of about 1 - 100, usually about 2 - 25 times the concentration of the test compound, and in those situations where the amount of test compound is unknown, times the average of the highest and lowest concentrations that can be estimated. For non-competitive assays, again the average of the highest and lowest concentrations that can be estimated can be used as an initial concentration and then the concentration optimized. The EA will be at least equal to the ED-conjugate and may be in substantial excess, usually being in substantial excess, generally about 10³ - 10⁶ -fold excess. The equations for defining the concentrations are found in U.S. Patent no. 4,378,428.

Where the target protein is one of the reagents, the concentration will be selected to optimize the complex rate change for a candidate compound having the desired affinity. Generally, one would wish to see a change of at least about 10% in the turnover of the substrate during the course of the assay, preferably at least about 15%. Since in many cases, the target protein will have a relatively weak binding affinity, as compared to antibodies, the full dynamic range of the complex will not be achievable. Generally, at least about 20% of the full dynamic range will be sufficient for the assay, preferably at least about 35% and more preferably at least about 50%. ("Full dynamic range" would be the range from the result in the absence of the target protein and at saturation of the ED-conjugate with the target protein.)

In carrying out the assay, mixtures of reagents and/or reagents and samples will be incubated for sufficient time for reactions to occur, usually being at least about 10 min, more usually at least about 15 min, to allow for sufficient binding to occur to provide a reliable readout. Addition of the substrate may occur at the time of combination of the reagents or after a sufficient incubation period. Numerous substrates are known that provide a detectable product, where the product can be detected by the absorption or emission of light, e.g. colorimetrically or fluorimetrically. Illustrative substrates include di-β-galactosidylfluorescein, β-galactodsidylumbelliferone, etc.

For convenience kits can be provided. For competitive assays *in vitro,* the ED conjugate, the target protein, substrate and EA are provided, where one or more of the components may be mixed, e.g. EA and substrate. For non-competitive assays, the kit would require the same constituents, except that the target protein would be present for a control. In the case of intracellular assays, the components would be modified, where the EA may be provided as a construct for expression of EA to be introduced into the cell or cells may be provided that are appropriately modified to provide EA in the cell. Generally, the kits would include an insert with instructions for performing the assay. The instructions may be printed or electronic, e.g. a CD or floppy disk. The kits find use in marketing the product and encouraging the use of the assay for research and commercial settings.

The following examples are intended to illustrate but not limit the invention.

### EXPERIMENTAL

### Materials and Methods

All Fmoc-protected amino acids were brought from Nova Biochem, San Diego, CA. The first amino acid loaded-PEG-resin and the Kaiser test reagents were bought from, Applied Biosystems, Foster city, CA. All other reagents were from Fisher Scientific and Sigma Chemicals, St Louis.

### Synthesis of ED fragment

The short ED fragments of β-galactosidase were synthesized using solid phase peptide chemistry manually employing Fmoc chemistry under N₂ stirring. The syntheses were carried out using low loaded PEG-Resins (loading 0.1-0.2 mmole/g resin) using appropriately loaded first Fmoc-amino acid residues. The couplings were performed using 4 equivalents of Fmoc-protected amino acids, 4 equivalents of benzotriazole-1-yl-oxy-tris-pyrrolidino-phosphonium hexafluorophosphate (PyBop), 4 equivalents of 1,Hydroxybenzotriazole (HOBt) and 8 equivalents of Diisopropylethylamine (DIPEA) reagent. The deprotection of the Fmoc group was carried out employing 20% piperidine in DMF. All couplings and deprotections were carried out in DMF. The couplings were monitored by Kaiser test at 100°C. In the case of secondary amino acids the efficiency of the coupling was monitored by chloranil test. The difficult peptide couplings were carried out for prolonged period of time in 0.1% TritonX-100 in DMF. After every 10mer- an aliquot of the resin was taken out, deprotected using neat Trifluoroacetic acid (TFA) containing a cocktail of scavengers, purified by RP-HPLC (C18, 300 A°) and the molecular weight corroborated by ESIMS/MALDI-MS. The final peptide was obtained by treating the peptide resin with neat TFA containing thioanisole, ethanedithiol, water and phenol for 5 hours at ambient temperature. The resin was filtered off and the filtrate concentrated in vacuo. Addition of anhydrous cold ether yielded the crude peptide as a white powder. It was finally purified under reverse phase conditions on a C18 column and the molecular weight corroborated by ESIMS.

### EA and ED complementation assays

The complementation kinetics for all the ED fragments was carried out in a multiwell plate. Serial dilutions of different enzyme fragments (starting range 1nM) with 1X EA (0.18 mg/ml) reagent for complementation were employed. The assay protocol was as follows: To 20 ul of assay buffer, 10 ul of ED (serial dilutions in ED dilution buffer) and 1X EA reagent were added. After two hours of incubation at room temperature 10 ul of fluorescence (0.4 mg/ml resorufin galactoside, Molecular Probes, Eugene, OR) or chemiluminescence (Galacton-Star/Emerald II, ABI, Foster City, CA) reagent was added. The plate was read using a Packard plate reader at 10 min time intervals for 2h. For fluorescence substrates an excitation wavelength of 530 nm and emission wavelength of 610 nm were used with PMT set at 1100V. The assay was performed in quadruplicate.
**1.** LQRRDWENPGVTQLNKLAAHPPFASWRNSEEARTDCPSQQL (41 mer) **(SEQ ID:NO 2)**
**2.** .IDPCASSNSLAWLQRRDWENPGVTQLNKLAAHPPF (36 mer) **(SEQ ID:NO 3)**
**3. S**PGIVIDPCASSNSLAVVLQRRDWENPGVTQLNKLAAHPPF **(40 mer) (SEQ ID:NO 4)**
**4. Q**SSPGNIDPCASSNSLAWLQRRDWENPGVTQLNKLAAHPPF (42 mer) **(SEQ ID:NO 5)**
**6. S**LAVVLQRRDWENPGVTQLNKLAAHPPFASWRNSEE**A (37 mer) (SEQ ID:NO 1)**
**7. S**LAVVLQRRDWENPGVTQLNKLAAHPPFASWRNSEEARTDCPSQQL **(46** mer) **(SEQ ID:NO 6)**
**8.** CSLAVVLQRRDWENPGVTQLNKLAAHPPFASWRNSEEARTDCPSQQL **(47 mer) (SEQ ID:NO 7)**
   **Enzyme fragment with purification and cleavage TAGS**
**9. AWRHPQFGG**SLAVVLQRRDWENPGVTQLNKLAAHPPFASWRNSEE**A (Strep Tag in 37 mer) (SEQ ID:NO 8)**
**10. HHHHHH**SLAVVLQRRDWENPGVTQLNKLAAHPPFASWRNSEE**A (6His Tag in 37 mer) (SEQ ID:NO9)**
**11. DYKDDDYK**SLAVVLQRRDWENPGVTQLNKLAAHPPFASWRNSEE**A** (Flag Tag in 37 mer) **(SEQ ID:NO 10)**
**12. HHHHHH**SLAVVLQRRDWENPGVTQLNKLAAHPPFASWRNSEE**ALVPRGS (6His Tag in 37 mer with Thrombin cleavage site at C-terminal) (SEQ ID:NO 11)**
13. 
HNHNHNHNHNHNSLAVVLQRRDWENPGVTQLNKLAAHPPFASWRNSEEALV
PRGS {6(His-Asn) Tag in 37 mer with thrombin cleavage site at C-terminal) (SEQ
ID:NO 12)
17. ED28 (90 mer) **(SEQ ID:NO 13)**

### Results:

**1.** The SAR studies of the native 90 mer (ED 28) DX 400090 demonstrated that 37 mer DX 400045 retained 45% of the complementation activity at ED concentration of 0.0123nM at 60 min. The rate of complementation was linear.
**2.** The complementation activity of the 45+1 mer was 72% at the same concentration
**3.** Addition of different purification tags as well as thrombin cleavage site to (37 mer) improved the complementation kinetics 5-10 %.
**4.** Other short EnzymeDonor fragments which had been prepared shorter than the 37mer (not shown) retained 3-6 % activity of the native 90 mer (SEQ ID:NO 17)
**5.** The lowest limit of detection for SEQ ID:NO 1 was in sub picomolar range indicating that it retained its sensitivity when compared to native 90 mer (SEQ ID:NO 17)

### Preparation of staurosporine-N-methylcarboxylic acid, methyl ester

To a vial of staurosporine (0.5 mg, 1.07 micromole) was added dimethylformamide (250 µL). An appropriately sized magnetic stirrer bar was added to the vial. To this were added methyl bromoacetate (4.8 mg, 31 micromole) and diisopropylethylamine (0.6 mL). Allowed the reaction mixture to stir overnight. High Performance Liquid Chromatography on a pharmaceutical C18 and a gradient of 0 (100% C) to 100 %D (buffer C: 0.1% TFA in HPLC water and buffer D: 0.1 % TFA in HPLC acetonitrile) analysis showed the reaction to be complete as one major product. Staurosporine-N-methylcarboxylic acid, methyl ester was purified by HPLC, and electro-spray mass spectroscopy (ESI-MS) confirmed the identity of the product (M+1 = 539). Lyophilized the product fraction overnight. This was used in the next synthetic step.

### Hydrolysis of staurosporine-N-methylcarboxylic acid, methyl ester

To the vial containing staurosporine-N-methylcarboxylic acid, methyl ester (∼ 0.5 - 0.6 mg) was added HPLC grade methanol (250 µL) and HPLC grade water (250 µL). An appropriately sized magnetic stirrer bar was added to the reaction vial. To the reaction was added sodium hydroxide (1N, 100 µL). The reaction mixture was stirred overnight. Analysis of the reaction mixture by HPLC showed one major product peak. The product was isolated by HPLC, and confirmed by ESI-MS (M+1 = 525). Lyophilized the reaction mixture overnight. This amount was used for the next step synthesis.

Preparation of staurosporine-N-(methylcarboxy-(2-maleimidoethyl)-amide); staurosporine-CM-MEA

To the vial of staurosporine-N-methylcarboxylic acid (∼ 0.5 mg, ∼ 0.9 micromole) was added HPLC grade dimethylformamide (125 µL). To this was added HPLC grade DMSO (125 µL). An appropriately sized magnetic stirrer was added to the reaction vial. To the reaction mixture was added maleimidoethylamine HCl (1.1 mg, 6.2 micromole). Prepared HBTU-HOBT solution by dissolving 95 mg of O-benzotriazol-1-yl-N, N, N', N', teramethyluronium hexafluorophosphate in 1 ml of a 0.5 M solution of 1-hydroxybenzotriazol hydrate in HPLC grade DMF. A solution of HBTU-HOBT (10 µL, ∼ 5 micromole) was added to the reaction mixture. Placed the reaction on ice. Initiated the reaction by adding diisopropylethylamine (1.1 µL, ∼ 6 micromole) to the reaction vial. Stirred the reaction for 5 min on ice. Analysis of the reaction mixture by HPLC showed a complete disappearance of the starting material. The product was purified by HPLC and confirmed by ESI-MS (M+1 = 647). The purified fraction was used directly in the conjugation.

### Preparation of (staurosporine-CM-MEA)₂-ED28 90 mer(DX 400090)

To a desalted solution of ED28 (∼ 0.25 mg, 26 nmole) in sodium phosphate buffer (0.160 mL) in an appropriately sized test tube was added a solution of purified staurosporine-CM-MEA from the previous step. Sodium phsphate buffer (100 mM, pH 8.5, 200-300 µL) was added to the reaction in order to adjust the pH to 7.0. Allowed the reaction to proceed for 1-2 hours. Purified the reaction mixture by HPLC (C4 protein column from Vydac, 1 x 25 cm, 5 micron particles). A step gradient of 20 % D (80 % C) to 60 % D was used in this purification. The conjugate elutes in 20 min at 4 mL / min flow rate. The conjugate was quantitated by UV-Vis spectroscopy, assuming ε₂₈₀ = 86,000 M⁻¹cm⁻¹ for this conjugate. The conjugate was confirmed by ESI-MS (M+1 = 11,082).

### Preparation of (staurosporine-CM-MEA)₂-47mer(DX400060)

To a desalted solution of DX400060 (∼ 0.25 mg, 26 nmole) in sodium phosphate buffer (0.160 mL) in an appropriately sized test tube was added a solution of purified staurosporine-CM-MEA from the previous step. Sodium phosphate buffer (100 mM, pH 8.5, 200-300 µL) was added to the reaction in order to adjust the pH to 7.0. Allowed the reaction to proceed for 1-2 hours. Purified the reaction mixture by HPLC (C18, 300 A column from Zorbax, 1 x 25 cm, 5 micron particles). A step gradient of 20 % D (80 % C) to 60 % D was used in this purification. The conjugate elutes in 24 min at 4 mL / min flow rate. The conjugate was quantitated by UV-Vis spectroscopy, assuming ε₂₈₀ = 80,000 M⁻¹cm⁻¹ for this conjugate. The conjugate was confirmed by ESI-MS (M+1 = 6641).

### Assay for staurosporine conjugates

Prepare serial dilutions of staurosporine (STA) or any other drug compound in assay buffer (ASB) containing 30 mM HEPES, pH=7.4, 10 mM MgCl₂, 0.4 mM EGTA, 20 mM NaCl, 0.01 % Tween-20, 0.1% Bovine beta-globuline. Pipette 10 uL of each dilution into 384-well plate. Do replicates. Prepare 36 uM peptide (substrate of a kinase) by diluting a stock solution (3.2 mM) with ASB.

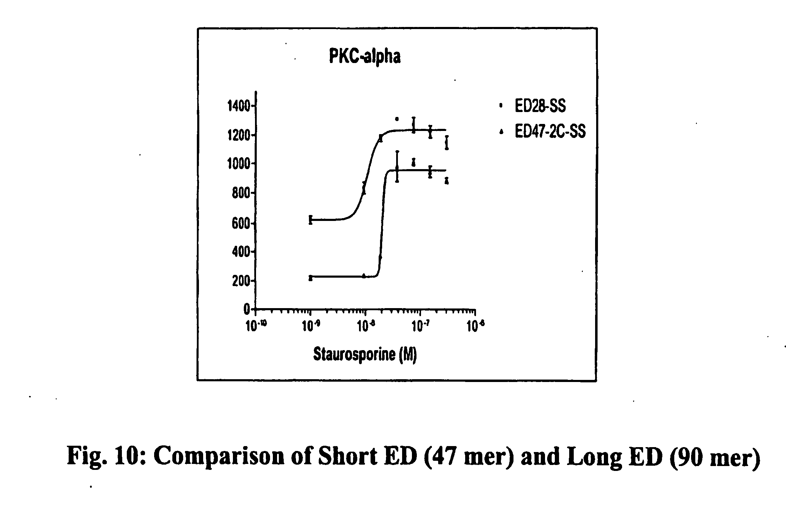

Prepare 4 x enzyme working solution (PKC). Prepare 0.25 nM ED₂₈-STA by diluted in1 to 1 mix of ASB and Enzyme donor dilution buffer (EDDB) containing 10 mM MES, pH=6.5, 200 mM NaCl, 10 mM EGTA, 2 mg/ml BSA fragments, 14.6 mM NaN₃). Mix equal amounts of a peptide, an enzyme working solution and ED-STA Pipette 30 uL of peptide/PKC/ED₂₈-STA mix onto the plate containing 10 uL of Staurosporine dilutions dispensed in each well. Tap the plate. Incubate 60 min at room temperature. Add 10 uL of 0.006 mg/ml Enzyme acceptor (EA) diluted with Enzyme acceptor dilution buffer (EADB) containing 100 mM PIPES, pH=6.83, 400 mM NaCl, 10 mM EGTA, 0.005% Tween-20, 150 mM NaOH, 10 mM Mg Acetate, 14.3 mM NaN₃. Add 15 uL of Galacton-Star/Emerald II (Chemiluminescent) substrate for beta-galactosidase (Tropix). Incubate 10-15 min. Read chemiluminescence within the first hour after addition of EA reagent. In a saturation binding study ED₂₈-STA had affinity of 20 nM at PKC concentration of 20 nM. In a competition experiments staurosporine is shown to be able to displace ED₂₈-STA with a potency of 16 nM. For further results, see Fig. 10.

### Progesterone derivatives: (DX - 200350)

Preparation of 4-Pregnan-21-ol-3, 20-dione-21-Maleimidoethylamino hemisuccinate.

4-Pregnan-21-ol-3, 20-dione-21-hemisuccinate (5.2 mg) was dissolved in 150 µL of anhydrous DMF and 350 µL of anhydrous DMSO. Maleimidoethylamine hydrochloride (5 mg) was dissolved in 200 µL of anhydrous DMF. The maleimide solution was slowly added to the amine solution and the reactions mixed by vortexing in an ice bath. To the reaction mixture, a solution of *O*-benzotriazol-1-yl-*N*,*N*,*N*',*N-*tetramethyluronium hexafluorophosphate and 1-hydroxybenzotriazole (0.25 M in DMF, 30 µL) and diisopropylethylamine (10 µL) was added. The product was purified by high performance liquid chromatography on a reversed phase column (C4).

Preparation of the conjugate of PL47DiCys (SEQ ID:NO 7) to 4-pregnan-21-ol-3, 20-dione-21-maleimidoethylamino hemisuccinate.

PL47DiCys (1 mg) was dissolved in 1 mL of Phosphate buffer (100mM with 2 mM EDTA, pH 6.5), 1 mL of anhydrous DMF, and 1 mL of HPLC grade acetonitrile. 4-Pregnan-21-ol-3, 20-dione-21-maleimidoethylamino hemisuccinate (0.6 mg) was dissolved in 1mL of phosphate buffer (100mM with 2 mM EDTA, pH 6.5), 0.5 mL of HPLC grade acetonitrile, and 1 mL of anhydrous DMF. The maleimide solution was slowly added to the PL47DiCys solution while mixing by vortex. After two hours, the product was purified by high performance liquid chromatography on a reversed phase column (C4). The identity of the product was confirmed by MS analysis (M⁺ = 6495).

Enzyme Fragment Complementation based Progesterone Receptor assay with ED[45+2] progesterone conjugate.

Solutions of progesterone (Steraloids Inc, Newport, RI ) at different concentrations were made by serial dilution of a 10mM stock solution in methanol. The serial dilutions were done in assay buffer (50 mM HEPES, 150 mM NaCl, and 0.1% Bovine Gamma Globulin (BGG), pH 7.4). To 1.0ul of progesterone (at different concentrations) was added 25ul of progesterone receptor and enzyme acceptor (15ul PR and 10ul of 1.8uM EA). The mixture was incubated in the microtiter plate for 60 minutes. 10ul of the ED-progesterone conjugate (0.5nM in ED dilution buffer composed of 10mM MES pH 5.5, 200mM NaCl, 10mM EGTA, 2mg/ml BSA fragments and 14.6mM NaN3) and 10ul of the chemiluminescent regent, Galacton Star with Emerald ( Applied Biosystems, Foster City, CA) were added together and the plate read on the Lumicount (Packard, Meridien, CT). The conjugate was found to have a dynamic range of about 10⁻⁶ - 10⁻⁸ with an EC₅₀ of 1.60 e^{-7.}

### Estrogen derivatives: (DX - 200350)

### Preparation of 1,3,5 (10)-estratriene-3, 17-diol-17-(2-Maleimidoethylamino) -hemisuccinate.

1,3,5 (10)-estratriene-3, 17-diol-17-hemisuccinate (5.2 mg) was dissolved in 150 µL of anhydrous DMF and 350 µL of anhydrous DMSO. Maleimidoethylamine hydrochloride (5 mg) was dissolved in 200 µL of anhydrous DMF. The maleimide solution was slowly added to the amine solution and the reactions mixed by vortexing in an ice bath. To the reaction mixture, a solution of *O*-benzotriazol-l-yl-*N*,*N*,*N*',*N*'-tetramethyluronium hexafluorophosphate and 1-hydroxybenzotriazole (0.25 M in DMF, 30 µL) and diisopropylethylamine (9.6 µL) was added. The product was purified by high performance liquid chromatography on a reversed phase column (C18).

Preparation of the conjugate of PL47DiCys (SEQ ID:NO. 7) to 1,3,5 (10)-estratriene-3, 17-diol-17-(2- Maleimidoethylamino) -hemisuccinate.

PL47DiCys (0.55 mg) was dissolved in 0.5 mL of Phosphate buffer (100mM with 2 mM EDTA, pH 6.5) and 0.5 mL HPLC grade acetonitrile. 1,3,5 (10)-Estratriene-3, 17-diol-17-(2- maleimidoethylamino) -hemisuccinate (0.35 mg) was dissolved in 1.5mL of phosphate buffer (100mM with 2 mM EDTA, pH 6.5) and 3 mL anhydrous DMF. The maleimide solution was slowly added to the PL47DiCys solution while mixing by vortex. After two hours, the product was purified by high performance liquid chromatography on a reversed phase column (C4). The identity of the product was confirmed by MS analysis (M⁺ = 6379).

### GTP-γ-S conjugates:

### Preparation of the GTP-γ-S-BMH derivative:

To a solution of GTP-γ-S (2 mg) in sodium phosphate (100 mM, pH 6.9, 1 mL) was added 200 µL of DMF. bis-Maleimidohexane(4 mg) was dissolved in minimum of DMF (∼ 200 µL). The maleimide solution was slowly added to the GTP-γ-S solution and mixed the reaction by vortexing. The product was purified by high performance liquid chromatography on a reversed phase column (C18). The molecular weight was corroborated by ESI-MS (M+=811).

### Preparation of the GTP-γ-S-BMOE derivative:

To a solution of GTP-γ-S (2 mg) in sodium phosphate (100 mM, pH 6.9, 1 mL) was added 200 µL, of DMF. bis-Maleimidoethane(4 mg) was dissolved in minimum of DMF (∼ 200 µL). The maleimide solution was slowly added to the GTP-γ-S solution and mixed the reaction by vortexing. The product was purified by high performance liquid chromatography on a reversed phase column (C18). The molecular weight was corroborated by ESI-MS (M+=755).

### Preparation of the conjugate of ED[45+2] to GTP-γ-S-BMH:

ED[45+2] (0.3 mg) was conjugated to GTP-γ-S-BMH (0.25 mg in 50 ml water) in 100mM sodium phosphate buffer (pH 6.9). After one hour the product was purified by high performance liquid chromatography on a reversed phase column (C18). The identity of the product was confirmed by ESI-MS analysis (M+= 7013)

### Preparation of the conjugate of ED[45+2] to GTP-γ-S-BMOE:

ED[45+2] (0.3 mg) was conjugated to GTP-γ-S-BMOE (0.25 mg in 50 ml water) in 100mM sodium phosphate buffer (pH 6.9). After one hour the product was purified by high performance liquid chromatography on a reversed phase column (C18). The identity of the product was confirmed by ESI-MS analysis (M+= 6901).p38 MAP kinase derivatives:

### Preparation of 4-(4-fluorophenyl)-5-(4-pyridyl)-2-(4,0-(carboxymethyloxy)phenyl)imidazole methyl ester (FHPI-cm-OMe).

To a solution of 4-(4-fluorophenyl)-5-(4-pyridyl)-2-(4-hydroxyphenyl)imidazole (FHPI (SB 202190), 2 mg) in methanol was added a solution of sodium methoxide in methanol (0.5N, 20 µL). Methanol was removed under high vacuum. To the residue was added anhydrous dimethylformamide and methylbromoacetate (2 µL). The reaction mixture was stirred for 30 minutes. The reaction was quenched with trifluoroacetic acid and the product was purified by high performance liquid chromatography on a reversed phase column (C18). The product peak was lyophilized and used in the next step.

Hydrolysis of 4-(4-fluorophenyl)-5-(4-pyridyl)-2-(4,O-(carboxymethyloxy)phenyl)imidazole methyl ester (FHPI-cm-OH).

The product of the previous step was dissolved in methanol (0.5 mL). To this was added water (0.5 mL) and sodium hydroxide solution (1N, 0.2 mL). The mixture was stirred for 2 hours. The reaction was quenched with trifluoroacetic acid and the product was purified by high performance liquid chromatography on a reversed phase column (C18). The product peak was lyophilized and used in the next step.

### Preparation of 4-(4-fluorophenyl)-5-(4-pyridyl)-2-(4,O-(carboxy-(2-maleimidoethyl)methyloxy)phenyl)imidazole (FHPI-cm-MEA)

FHPI-cm-OH (1.1 mg) was dissolved in dimethylsulfoxide (100 µL) and dimethylformamide (100 µL). To this were added 2-(1H-benzotnazole-1-yl)-1,1,3,3-tetramethyluronium hexaflurophosphate (HBTU, 3.2 mg), maleimidoethylamine hydrochloride (MEA.HCL, 1.5 mg) and diisopropylethylamine (DIEA, 2.0 µL). The reaction was quenched with trifluoroacetic acid and purified by high performance liquid chromatography on a reversed phase column (C18). Analysis of the product by electro-spray ionization (p⁺) mass spectroscopy positively identified the product (M⁺+1 = 512).

Conjugation of 4-(4-fluorophenyl)-5-(4-pyridyl)-2-(4,O-(carboxy-(2-maleimidoethyl)methyloxy)phenyl)imidazole to ED [45+2].

ED[45+2] (0.25 mg) was conjugated to 4-(4-fluorophenyl)-5-(4-pyridyl)-2-(4,0-(carboxy-(2-maleimidoethyl)methyloxy)phenyl)imidazole (0.3 mg) in sodium phosphate buffer (100 mM, pH = 7.0). After one hour the products were purified by high performance liquid chromatography on a reversed phase column (C18). The product was positively identified by MALDI-TOF analysis (M⁺ = 6413).

### Structures of the derivatives:

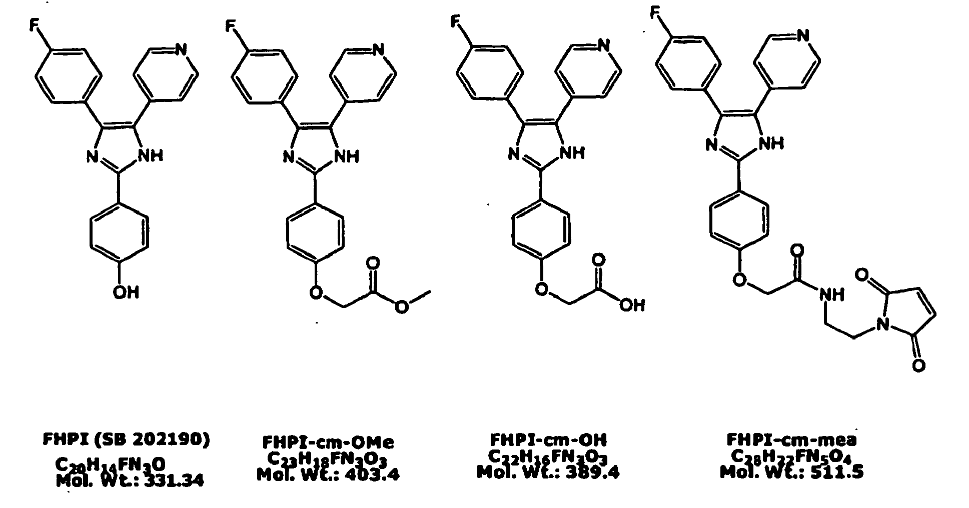

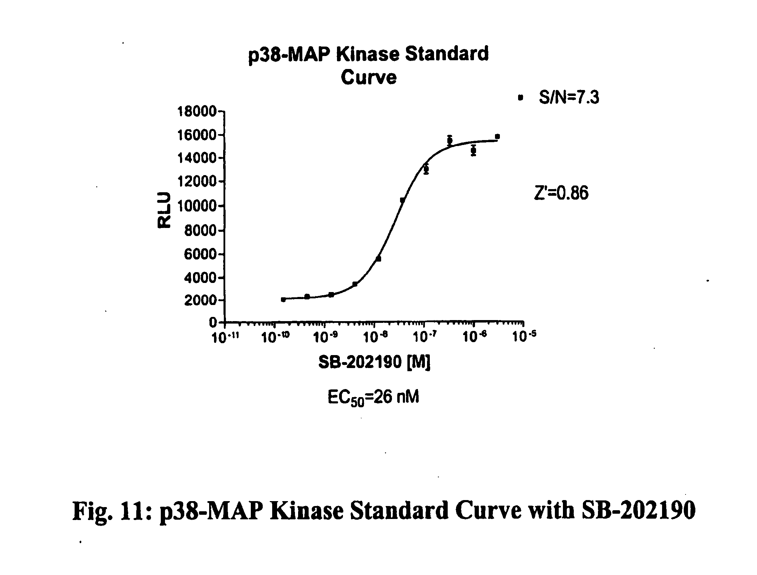

Enzyme Fragment Complementation based competition assay with FHPI (SB202190) and ED[45+2] FHPI-cm-mea conjugate (See Figure 11). Solutions ofFHPI (SB 202190, Calbiochem, CA) at different concentrations were made by serial dilution of a 15mM stock solution in DMSO. The serial dilutions were done in assay buffer. 20µl of the FHPI compound was incubated with 10 ul of enzyme acceptor (EA, 450nM) and the Kinase (40nM, p38-GST, Calbiochem, CA), for 30-60 minutes. The ED[45+2] FHPI-cm-mea conjugate (10ul, 0.25nM) was added and the reaction mixture incubated for another 30minutes. 10µl of the chemiluminescent substrate, Galacton Star with Emerald (Applied Biosystems, Foster City, CA) was added and read after 30-60 minutes. The assay was done in a microtiter plate and read on a Lumicount (Packard, Meridien, CT). The EC50 of FHPI (SB202190) was found to be 26nM.

20ul of membrane protein (10ug from CHO-M1) in a binding buffer (50mM HEPES, 20mM NaCl, 10mM MgCl2, with 1mM DTT or 0.01% CHAPS) was incubated with 10ul of enzyme donor-GTP gamma-S conjugate at various concentrations for 60 minutes at ambient temperature. 10ul of enzyme acceptor (0.18uM) and 15ul of the chemiluminescent reagent, Galacton Star with Emerald plus (Applied Biosystems, Foster city, CA) added and the microtiter plate read on the Lumicount (Packard, Meridien, CT). The readings are tabulated below with ED46mer used as control. Open and Close refer to free and bound enzyme donor conjugate to enzyme acceptor. Percentage inhibition is calculated by (open-close)/open readings.

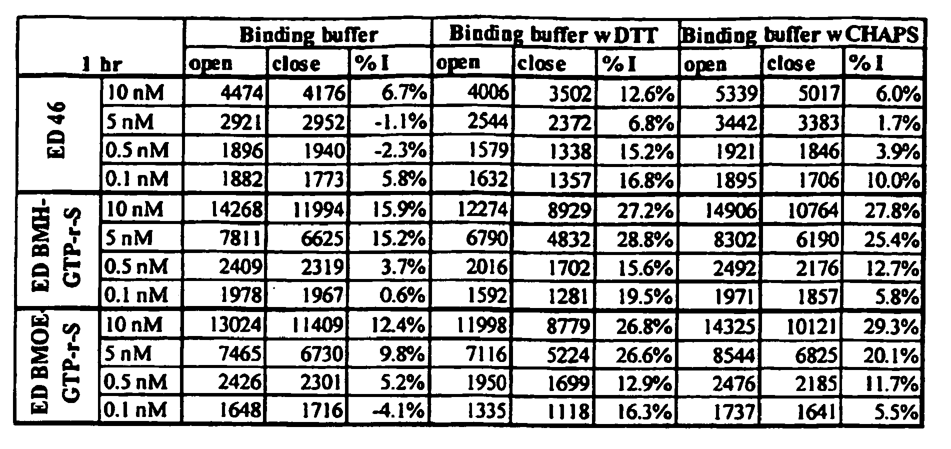

In order to test if the inhibition was capable of being modulated. The experiments were performed by incubating the membrane preparation with GTPgamma-S (10ul of 10uM). Higher concentrations of enzyme donor together with another detergent saponin were tried to enhance signal and minimize non-specific binding.

10ul of membrane protein (10ug from CHO-M1) in a binding buffer (50mM HEPES, 20mM NaCl, 10mM MgCl₂, with 0.01% CHAPS or 0.01% saponin) was incubated with 10ul of GTP gamma-S for 30 minutes at room temperature 10ul of the enzyme donor conjugate at various concentrations was added and the mixture incubated for 60 minutes at ambient temperature. 10ul of enzyme acceptor (0.45uM) and 15ul of the chemiluminescent reagent, Galacton Star with Emerald plus (Applied Biosystems, Foster city, CA) added and the microtiter plate read on the Lumicount (Packard, Meridien, CT). The readings are tabulated below with ED46mer used as control. Open and close refer to free and bound enzyme donor conjugate to enzyme acceptor. Percentage inhibition is calculated by (open-close)/open readings and is in the absence of GTP-gamma-S.

In order to test if the inhibition was capable of being modulated. The experiments were performed by incubating the membrane preparation with GTPgamma-S (10ul of 10uM). Higher concentrations of enzyme donor together with another detergent saponin were tried to enhance signal and minimize non-specific binding. 10ul of membrane protein (10ug from CHO-M1) in a binding buffer (50mM HEPES, 20mM NaCl, 10mM MgCl₂, with 0.01% CHAPS or 0.01% saponin) was incubated with 10ul of GTP gamma-S for 30 minutes at room temperature 10ul of the enzyme donor conjugate at various concentrations was added and the mixture incubated for 60 minutes at ambient temperature. 10ul of enzyme acceptor (0.45uM) and 15ul of the chemiluminescent reagent, Galacton Star with Emerald plus (Applied Biosystems, Foster city, CA) added and the microtiter plate read on the Lumicount (Packard, Meridien, CT). The readings are tabulated below with ED46mer used as control. Open and close refer to free and bound enzyme donor conjugate to enzyme acceptor. Percentage inhibition is calculated by (open-close)/open readings and is in the absence of GTP-gamma-S.

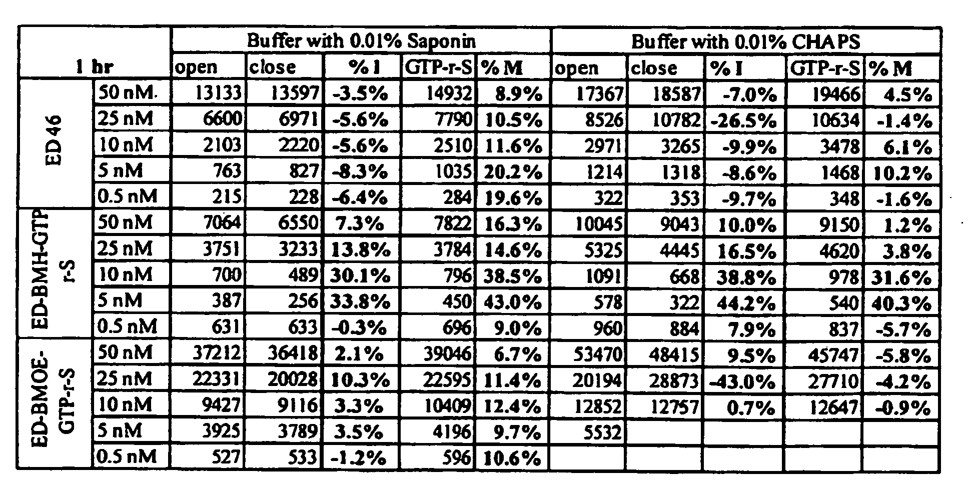

It is evident from the above results that a short ED can provide desired levels of sensitivity for use in assays, for the determination of analytes, for following events intracellularly, and the like. By being short enough to be readily synthesized, flexibility is provided for having both polypeptide and non-amino acid substitutions. In this way, one can study a variety of reactions resulting in cleavage, degradation, complex formation, translocation, and the like, where the short ED diminishes the likelihood of interference with these processes, while providing sufficient sensitivity for monitoring these events.

## Claims

1. A method for determining a binding event of a protein receptor with a ligand, wherein said protein receptor is other than an antibody or polyvalent fragment thereof and is either an enzyme or is a membrane receptor wherein the membrane receptor is a component of an intact cell or of a lysed cell, said method comprising:
employing as enzyme donor (ED) a β-galactosidase fragment of from 36 to 50 amino acids;
combining a conjugate of said ED and of a ligand complementary to said protein receptor with said protein receptor; and
measuring the amount of complex formation of said ED with an enzyme acceptor fragment (EA) by means of a detectable product provided by a substrate.

2. A method according to claim 1, wherein said protein receptor is an enzyme.

3. A method according to claim 1, wherein said protein receptor is a membrane receptor.

## Patentansprüche

1. Verfahren zur Bestimmung einer Bindung eines Proteinrezeptors mit einem Liganden, wobei der genannte Proteinrezeptor etwas anderes als ein Antikörper oder ein polyvalentes Fragment daraus ist und entweder ein Enzym oder ein Membranrezeptor ist, wobei der Membranrezeptor ein Bestandteil einer intakten oder einer lysierten Zelle ist, das genannte Verfahren umfasst:
Einsetzen einer β-Galactosidasefragmentes aus 36 bis 50 Aminosäuren als Enzymdonor (ED);
Kombinieren eines Konjugats des genannten ED und eines zu dem genannten Protein komplementären Liganden mit dem genannten Proteinrezeptor; und
Messen des Anteils der Komplexbildung des genannten ED mit einem Enzymrezeptorfragment (EA) mittels eines detektierbaren Produkts, das durch ein Substrat bereitgestellt wird.

2. Verfahren nach Anspruch 1, wobei der genannte Proteinrezeptor ein Enzym ist.

3. Verfahren nach Anspruch 2, wobei der genannte Proteinrezeptor ein Membranrezeptor ist.

## Revendications

1. Méthode pour déterminer un événement de liaison d'un récepteur protéique avec un ligand, dans laquelle ledit récepteur protéique est autre qu'un anticorps ou un fragment polyvalent de celui-ci et est soit une enzyme soit un récepteur membranaire dans lequel le récepteur membranaire est un composant d'une cellule intacte ou d'une cellule lysée, ladite méthode comprenant :
l'utilisation comme donneur enzymatique (ED),
d'un fragment de β-galactosidase de 36 à 50 acides aminés ;
la combinaison d'un conjugué dudit ED et d'un ligand complémentaire dudit récepteur protéique avec ledit récepteur protéique ; et
la mesure de la quantité de formation d'un complexe dudit ED avec un fragment accepteur enzymatique (EA) au moyen d'un produit détectable fourni par un substrat.

2. Méthode de la revendication 1, dans laquelle ledit récepteur protéique est une enzyme.

3. Méthode de la revendication 1, dans laquelle ledit récepteur protéique est un récepteur membranaire.
